(19)

Europäisches Patentamt
European Patent Office
Office européen des brevets

(11) **EP 2 267 146 A1**

(12) **EUROPEAN PATENT APPLICATION**
published in accordance with Art. 153(4) EPC

(43) Date of publication:
**29.12.2010 Bulletin 2010/52**

(21) Application number: **09721139.5**

(22) Date of filing: **12.03.2009**

(51) Int Cl.:
*C12P 21/02* *(2006.01)*       *C12N 9/10* *(2006.01)*
*C12Q 1/48* *(2006.01)*       *C12Q 1/52* *(2006.01)*

(86) International application number:
**PCT/JP2009/054792**

(87) International publication number:
**WO 2009/113628 (17.09.2009 Gazette 2009/38)**

(84) Designated Contracting States:
**AT BE BG CH CY CZ DE DK EE ES FI FR GB GR
HR HU IE IS IT LI LT LU LV MC MK MT NL NO PL
PT RO SE SI SK TR**
Designated Extension States:
**AL BA RS**

(30) Priority: **14.03.2008 JP 2008066765
18.11.2008 JP 2008294890**

(71) Applicants:
• **Ajinomoto Co., Inc.**
**Tokyo 104-8315 (JP)**
• **Amano Enzyme Inc.**
**Nagoya-shi**
**Aichi 460-8630 (JP)**

(72) Inventors:
• **MIWA, Noriko**
**Kawasaki-shi**
**Kanagawa 210-8681 (JP)**
• **SHIMBA, Nobuhisa**
**Kawasaki-shi**
**Kanagawa 210-8681 (JP)**

• **NAKAMURA, Mina**
**Kawasaki-shi**
**Kanagawa 210-8681 (JP)**
• **SUZUKI, Eiichiro**
**Kawasaki-shi**
**Kanagawa 210-8681 (JP)**
• **YOKOYAMA, Keiichi**
**Kawasaki-shi**
**Kanagawa 210-8681 (JP)**
• **NAKAGOSHI, Hiroyuki**
**Kawasaki-shi**
**Kanagawa 210-8681 (JP)**
• **HIROSE, Fumiyuki**
**Kawasaki-shi**
**Kanagawa 210-8681 (JP)**
• **SATO, Hiroaki**
**Kawasaki-shi**
**Kanagawa 210-8681 (JP)**

(74) Representative: **Strehl Schübel-Hopf & Partner**
**Maximilianstraße 54**
**D-80538 München (DE)**

(54) **METHOD OF DENATURING PROTEIN WITH ENZYMES**

(57)    Provided are: a method of denaturing a protein by treating the protein with both of protein glutaminase and transglutaminase; a food containing a protein having been denatured with these enzymes; an enzyme preparation for denaturing a protein which comprises these enzymes; and a method of establishing an optimum quantitative ratio of protein glutaminase to transglutaminase both of which are to be added to a protein. A protein is denatured by adding protein glutaminase and transglutaminase to the protein substantially at the same timing, or adding protein glutaminase to the protein before the transglutaminase acts on the protein, or controlling the quantitative ratio of protein glutaminase to transglutaminase, by which a protein is treated, to a definite level. The optimum quantitative ratio of protein glutaminase to transglutaminase, both of which are to be added to a protein, is determined based on the NMR signal intensity of the protein labeled with isotope-labeled ammonium or the like.

FIG. 1

**Description**

TECHNICAL FIELD

[CROSS-REFERENCE TO RELATED APPLICATION]

This application is based upon and claims the benefit of the priority of Japanese patent applications No.2008-066765 filed on March 14, 2008 and No.2008-294890 filed on November 18, 2008, the disclosures of which are incorporated herein in their entirety by reference thereto. The present invention relates to a method of modifying a protein by treating the protein with both of transglutaminase and protein glutaminase, which are enzymes for modifying a glutamine residue in protein, and causing two enzyme reactions. The present invention also relates to a food containing a protein having been modified by the method, an enzyme preparation for modifying a protein which contains both of the protein glutaminase and transglutaminase in a specified ratio, and a method of establishing the optimum quantitative ratio of protein glutaminase to transglutaminase both of which are to be added to substrate protein.

BACKGROUND

Transglutaminase (also referred to as "TG" hereinafter) is an enzyme to catalyze an acyl transfer reaction between a γ-carboxyamide group of a glutamine residue and a primary amine in proteins and peptides. The TG acts on ε-amino group of lysine residue in protein as an acyl receptor, which results in cross-linking polymerization reaction of proteins. When a primary amine does not exist, water functions as an acyl receptor and the TG catalyzes deamidation reaction to transform a glutamine residue to a glutamic acid residue. The cross-linking reaction of proteins is mainly utilized for food treatment. Nowadays various TG preparations are developed for various foods such as kamaboko, ham, sausage, noodles, bean curd and bread by utilizing their characteristics of adding or improving of gel formability of protein, gelation of unheated protein or adhesiveness.

On the other hand protein glutaminase (also referred to as "PG" hereinafter) is an enzyme that has a function of deamidation of an amide group of a glutamine residue in protein. The function is disclosed in detail in Patent Documents 1 and 2 and Non-Patent Document 1, etc. According to these documents, PG acts on the amide group in protein directly, which results in a transformation of glutamine residue into glutamic acid residue, and therefore an increasing of negative charge, an increasing of electrostatic repulsive force, a decreasing of isoelectric point, an increasing of hydration capability, etc. of protein occur because a carboxylic group is produced. As a result, improvements of functionalities such as an increasing of solubility and dispersion characteristic in water, an improvement of emulsification ability and emulsion stability, and the like are rendered and the broader usages of protein can be expected.

As explained above, both of TG and PG are known as industrially useful enzymes; however, few or no concrete examples are reported to obtain new added values by combined use of these enzymes. Rather than that, Patent Document 1 discloses an example of use of PG as a TG reaction control agent and Non-Patent Document 2 describes that a cross-linking reaction by TG does not proceed when PG and TG coexist. Further, knowledge at present with regard to differences of reactivity of TG and PG will be explained in detail. A target of both enzymes in substrate protein is a glutamine residue, which is common to both of the enzymes. However, a specificity of PG to individual glutamine residue in protein is broader than that of TG. The reason is estimated that the TG requires ε-amino group of lysine residue as well as a glutamine residue, on the other hand, the PG requires only water which exists abundantly in a reaction environment other than a glutamine residue. For example, the PG has a far higher catalytic efficiency of reactivity to glutamine residue in casein than that of TG by judging from Km value and kcat value. When both TG and PG coexist, the reaction with PG occurs under priority to TG and the cross-linking reaction does not proceed because deamidated glutamine residue is no longer a target substrate for TG. Actually, by an experiment of adding PG during a cross-linking polymerization of casein by TG, it was proved that the polymerization of casein was ceased at the same time of addition of PG. Moreover, it is confirmed that casein fully deamidated by PG is no longer a target substrate of TG, that is, no longer cross-linking-polymerized (Non-Patent Document 2). In addition, Y. S. Gu, et al. searched reactivity of the enzyme to an α-lactalbumin and reported that four of six glutamine residues were deamidated (Non-Patent Document 3). On the other hand, they reported that substrate specificity of PG was broader than that of TG because actinomycete TG acted only on glutamine 54 which was one of the four glutamine residues. Patent Document 1 refers to a possibility that a known TG inhibitor such as an EDTA or ammonium chloride, which is not desirable for food additives, may be replaced by PG because the PG can cease the TG reaction at an appropriate point of time by taking advantage of high reactivity of PG to glutamine residue.

However, no suggestion of combined use method of TG and PG has been made until now from the view point other than the stopping of TG reaction by PG. Furthermore, we cannot know from prior arts a condition for obtaining desirable target effects by treating substrate protein with both of TG and PG, in more detail, a condition that the TG reaction is not ceased by PG and the TG reaction and its modifying effects is maintained even when PG coexists. Also, no detailed method is disclosed for effective use of both enzymes such that, for example, what kinds of effects will be obtained when various kinds of substrate proteins are treated with TG and PG at various ratios. Therefore, at the present time, it is necessary to try and fail to find the best reaction conditions for each usage when developing protein products modified by TG and PG, which is regarded as completely a new attempt.

The enzyme reaction differs in its reaction amount and its degree of effect by various factors such as a treating time, temperature, amount of added enzyme, sort of substrate, condition, substrate specificity, and the like and it is difficult to use the two enzymes of common substrate efficiently. Therefore, it needs much time and effort for a developer or manufacturer to determine manufacturing conditions of intended food for each use. That is because an enzyme reaction differs in its reaction amount and its degree of effect by various factors such as a treating time, temperature, amount of added enzyme, sort of substrate, condition, substrate specificity, and the like, it is necessary to research every combination when attempting to use two enzymes at the same time. Therefore, a method to use two enzymes of common substrate efficiently is desired.

At present, a method to use NMR is known for searching substrate specificity of TG (Patent Document 3). This is a method to chase a TG reaction by treating any protein with TG under existence of $^{15}$N labeled ammonium chloride and detecting the $^{15}$N labeled nitrogen of carboxyamide of glutamine residue as a substrate of TG using NMR. The method can analyze both reactivity and substrate specificity at the same time using a protein substrate. However, Patent Document 3 does not disclose that the nitrogen of carboxyamide of glutamine residue that react with PG is labeled with $^{15}$N.

[Patent Document 1] Japanese Patent Kokai Publication No. JP-P2000-50887A
[Patent Document 2] Japanese Patent Kokai Publication No. JP-P2001-218590A
[Patent Document 3] Japanese Patent Kokai Publication No. JP-P2002-332295A
[Non-Patent Document 1] Yamaguchi et al., Eur.J.Biochem. Vol.268, 2001, pp.1410-1412
[Non-Patent Document 2] Newest Technology and Application of Food Enzyme Chemistry-Prospect of Proteomics-, CMC Publishing Co., Ltd., pp146-153
[Non-Patent Document 3] Y.S.Gu et al., J. Agric. Food Chem. Vol.49, 2001, pp.5999-6005

DISCLOSURE OF THE INVENTION

PROBLEMS TO BE SOLVED BY THE INVENTION

The entire disclosures of the above Patent Documents 1 to 3 and Non-Patent Documents 1 to 3 are incorporated herein by reference thereto. The following analysis is given by a view of the present invention.
It is an object of the present invention to provide a method of modifying a protein by treating the protein with both of protein glutaminase and transglutaminase, a food containing a protein modified with both of the enzymes, an enzyme preparation containing both of the enzymes for modifying a protein, and a method of easily establishing the optimum quantitative ratio of protein glutaminase to transglutamoinase.

MEANS TO SOLVE THE PROBLEMS

As a result of inventors' continuous research based on the prior art above explained, although it was estimated that a coexistence effect of PG and TG is difficult to obtain because TG does not work under the presence of PG, the inventors have found that both enzymes act on a substrate protein under certain conditions and a coexistence effect of PG and TG can be obtained even when both of the enzymes coexist. In addition, the inventors have found the fact that PG has a function of catalyzing an exchanging reaction of glutamine residue in a protein with an ammonium salt as well as catalyzing a deamidation of the glutamine residue as the function of TG. That is, carboxylamide nitrogen of glutamine residue on which the TG and PG act is labeled with $^{15}$N. Therefore, the difference of reactivity can be determined from estimation of labeled rate with $^{15}$N by NMR signal intensity of $^{1}$H-$^{15}$N HSQC measurement, for example, that detects labeled $^{15}$N. The inventors have also found that an existence ratio of TG to PG has a good correlation with NMR signal intensity ratio for various proteins. Moreover, the inventors have found that a quantitative ratio or activity ratio of PG to TG by which a signal intensity ratio of PG to TG (referred to as "PG/TG signal intensity ratio" hereinafter) shows within the range from 0.2 to 3.0 is the condition by which the TG reaction and its modifying effect can be maintained without ceasing of the TG reaction by PG in spite of coexistence of PG. That is, the inventors have found that a condition for obtaining coexistence effect of both enzymes can be determined easily by treating a substrate with TG or PG under existence of $^{15}$N labeled ammonium and comparing substrate specificity of each enzyme using NMR. The inventors have found the merits of co-treatment with TG and PG at the same time and succeeded to establish a method of determining the condition easily and practically. The present inventions are described as follows.

(1) A method of modifying a protein by treating the protein with both of protein glutaminase and transglutaminase in which a timing of adding protein glutaminase to a protein is essentially the same as a timing of adding transglutaminase to the protein or before the transglutaminse acts on the protein.

(2) The method of (1) described above in which a ratio by activity of the protein glutaminase to the transglutaminase, both of which are to be added to the protein, is described as the protein glutaminase: the transglutaminase =0.05 to 3:1.

(3) The method of (1) or (2) described above in which a ratio by weight of the protein glutaminase to the transglutaminase, both of which are to be added to the protein, is described as the protein glutaminase: the transglutaminase =0.01 to 0.7:1.

(4) The method of one of (1) to (3) described above in which a ratio by NMR signal intensity of the protein glutaminase to the transglutaminase, both of which are to be added to the protein, is described as the protein glutaminase: the transglutaminase = 0.2 to 3.0:1.

(5) The method of one of (1) to (4) described above in which the protein is one or more selected from a group consisting of a milk protein, whey protein, soybean protein, wheat gluten, plasma, muscle protein, collagen and gelatin.

(6) A food comprising a protein modified by one of the method (1) to (5) described above.

(7) An enzyme preparation for modifying a protein comprising protein glutaminase and transglutaminase in which a ratio by activity of the protein glutaminase to the transglutaminase in the enzyme preparation is described as the protein glutaminase: the transglutaminase = 0.05 to 3:1.

(8) An enzyme preparation for modifying a protein comprising protein glutaminase and transglutaminase in which a ratio by weight of the protein glutaminase to the transglutaminase in the enzyme preparation is described as the protein glutaminase: the transglutaminase = 0.01 to 0.7:1.

(9) A enzyme preparation for modifying a protein comprising protein glutaminase and transglutaminase in which a ratio by NMR signal intensity of the protein glutaminase to the transglutaminase in the enzyme preparation is described as the protein glutaminase: the transglutaminase = 0.2 to 3.0:1.

(10) A method of establishing the optimum ratio of protein glutaminase to transglutaminase, both of which are to be added to a protein, comprising a step of treating a protein with transglutaminase and protein glutaminase separately under presence of an isotope-labeled ammonium salt, a step of labeling a functional group of glutamine residue of the protein with the isotope, and a step of measuring an NMR signal intensity of the protein.

EFFECT OF THE INVENSION

The present invention provides a new method of modifying a protein by which an effect of modifying a protein, which is a combined use effect of TG and PG and different from that when the TG or PG is used by alone, can be obtained. In detail, characteristics of a protein exerting to a food containing the protein are modified and an oral sensation (hardness, smoothness, and the like) of the food is improved. The present invention also provides a food containing a protein having new characteristics that can be obtained by the modifying method. Also the present invention provides a simple method of establishing the optimum condition (condition of adding) for obtaining the effect above explained by treating a protein with both of TG and PG that act on a substrate of the protein.

BRIEF DESCRIPTION OF THE DRAWINGS

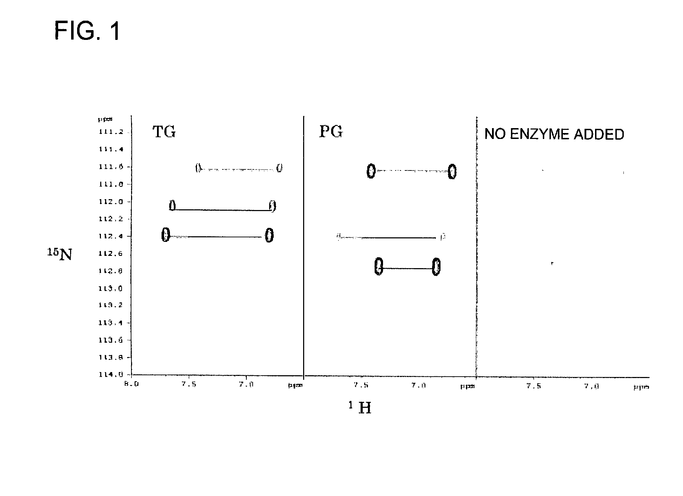

Fig. 1 shows a $^1$H-$^{15}$N HSQC NMR spectrum of $\alpha$-Lactalbumin in the presence of $^{15}$NH$_4$Cl (Experimental example 1).

PREFERRED MODES FOR CARRYING OUT THE INVENTION

Transglutaminase, which the present invention utilizes, is widely used for manufacturing foods such as gelatin, cheese, yoghurt, bean curd, steamed fish paste, ham, sausage, noodles, etc. and for improving quality of meat, and the like (Japanese Patent Kokai Publication No. JP-S64-27471A <Reference 1>). Beside that, TG is an enzyme used for various industrial purposes such as a material for a microcapsule that is stable in heat, manufacturing a carrier of an immobilized enzyme, and the like. TG catalyzes an acyl transfer reaction of a $\gamma$-carboxyamide group of glutamine residue in a peptide chain of a protein molecule. When TG acts on $\epsilon$-amino group of lysine residue of a protein molecule as an acyl receptor, an $\epsilon$-($\gamma$-glutamic acid)-lysine bonding is formed in and inter protein molecules. The disclosure of Reference 1 is incorporated herein in its entirety by reference thereto.

There are two types of TG, calcium independent type and calcium dependent type, and both types of TG may be used for the present invention. TG obtained from microorganisms such as an actinomycete or Bacillus subtilis, etc. may be an example as the former (for example, see Japanese Patent Kokai Publication No. JP-S64-27471A <Reference 2>). Examples as the latter are exemplified as that obtained from a liver of a guinea pig (for example, see Japanese Patent Kokoku-examined Publication No. JP-H01-50382B <Reference 3>), TG of human epidermal keratin cell (Phillips, M. A. et al. (1990) Proc. Natl. Acad. Sci. U.S.A., 87, 9333. <Reference 4>), human blood coagulation factor XIII (Ichinose, A. et al. (1990) Biochemistry 25, 6900. <Reference 5>), ones obtained from microorganisms such as an oomycete, etc., ones obtained from animal such as cattle blood or pig blood, etc., ones obtained from fish such as a salmon or porgy, etc. (for example, Nobuo Seki, et al., Nippon Suisan Gakkaishi (Bulletin of Japanese Society of Fisheries Science), vol. 56, pp125-132, 1990. <Reference 6>), ones obtained from an oyster, and the like. Moreover, TG produced by genetic recombination (for example, Japanese Patent Kokai Publication No. JP-H01-300889A <Reference 7>, Japanese Patent Kokai Publication No. JP-H06-225775A <Reference 8>, and Japanese Patent Kokai Publication No. JP-H07-23737A <Reference 9>), etc. can be applicable. Any type of TG can be applicable to the present invention and not limited by its origin or production method. In a case where an enzyme reaction in a solvent containing calcium is not desirable due to characteristics of a protein labeled with an isotope, the calcium non-dependent TG is preferable for such a protein. For example, (MTG) obtained from microorganisms (Japanese Patent Kokai Publication No. JP-S64-27471A <Reference 10>, for example) or the like satisfies the condition and therefore, it may be the best choice at the present time. They are, for example, obtained from Streptoverticillium griseocarneum, IFO 12776, Streptoverticillium cinnamoneum sub sp. Cinnamoneum, IFO 12852, Streptoverticillium mobaraense, IFO 13819, and the like. Transglutaminse obtained from Streptoverticillium mobaraense may be referred to as MTG hereinafter. The disclosures of above References 2 to 10 are incorporated herein in their entirety by reference thereto.

An activity unit of TG used in the present invention is determined and defined as follows. Benzyloxycarbonyl-L-Glutamylglycine (Z-Gln-Gly) and hydroxylamine as substrates are reacted with TG, and produced hydroxamic acid is converted into an iron complex in the presence of trichloroacetate and the amount of the iron complex is determined by absorbance at 525nm. A calibration curve is obtained from the amount of the hydroxamic acid and an amount of enzyme that produces 1 μmol of hydroxamate per 1 min is defined as 1 unit of activity unit. The detailed method of the measurement is already disclosed (for example, Japanese Patent Kokai Publication No. JP-S64-27471A <Reference 11>, etc.). The disclosure of Reference 11 is incorporated herein in its entirety by reference thereto.

PG used in the present invention acts directly to an amide group of a protein and causes deamidation without hydrolysis of peptide bonding and cross-linking of the protein. A kind of PG is not limited to the extent that the PG possesses such a function. Patent Documents 1 and 2 disclose such kinds of enzymes but not limited to those enzymes. PG prepared from a culture liquid for microorganism that produces the PG can be used. The microorganism for preparation of PG is not particularly limited and microorganisms such as Chryseobacterium, Flavobacterium and Empedobacter are illustrated.

Publicly known separation and purification methods of protein (such as centrifuging, UF concentration, salting-out, various kinds of chromatography with ion-exchanging resin, etc.) can be used for a preparation method of PG from a culture liquid for microorganism. For example, culture liquid is centrifuged to separate bacterial cells and then salting-out and chromatography, etc. may be combined to obtain target enzymes. When collecting enzymes from bacterial cells, the bacterial cells are crushed by pressure processing or supersonic processing, for example, and then separated and purified as described above to obtain target enzymes. Bacterial cells may be recovered from culture liquid by filtration or centrifuge, etc. prior to the processing steps above explained such as crushing of bacterial cells, separation and purification. The enzymes may be powdered by drying such as freeze drying or vacuum drying, etc. and appropriate diluent or drying auxiliary agent may be added at the drying step.

The activity unit of PG of the present invention can be measured by an improved method of a method of Patent Document 1 as follows.

(1) 10 μl of water solution containing PG is added to 100 μl of 176 mM phosphate buffer (pH 6.5) containing 30 mM of Z-Gln-Gly, incubated 10 minutes at 37 degrees C and then the reaction is ceased by adding 100 μl of 12% TCA solution. At this time the solution is diluted with 20 mM phosphate buffer (pH 6.0) such that the concentration of enzyme becomes 0.05 mg/ml.

(2) The solution is centrifuged (12000 rpm, 4 degrees C, 5 minutes) and $NH_3$ in the supernatant is measured with F-kit-Ammonia (Roche). The method is as follows.

(3) 10 μl of the supernatant and 190 μl of 0.1M triethanolamine buffer (pH 8.0) are added to 100 μl of liquid reagent II (attachment of the F-kit) and settled for 5 minutes at room temperature. After that an absorbance (E1) at 340 nm is measured using 100 μl of the solution. 1.0 μl of reagent III (glutamate dehydrogenase) is added to the remaining 200 μl of the solution and settled for 20 minutes at room temperature and then an absorbance (E2) at 340 nm is measured using the 200 μl of the solution. An ammonia concentration in the reaction solution is determined using a calibration curve indicating the relation between an ammonia concentration and variation of absorbance (at 340 nm) prepared using an ammonia standard solution attached to the F-kit.

(4) Concentration of protein is measured using protein assay CBB (Coomassie Brilliant Blue) solution (Nacalai Tesque) at 595 nm wavelength. BSA (Pierce) is used as a standard.

(5) Activity of PG is calculated by the following formula.

Relative activity (U/mg)=(ammonia concentration in reaction solution (μmol/ml) x volume of reaction solution (ml) x dilution rate of enzyme)/(enzyme amount (ml) x concentration of protein (mg/ml) x reaction time (min))

According to the present invention, a timing of adding PG to a protein is essentially the same as a timing of adding TG to the protein or before the TG acts on the protein. Therefore, the method disclosed in Patent Document 1 of ceasing TG reaction by adding PG is not included in the present invention because the timing of adding PG to a protein is after the TG acts on the protein. It should be noted that "PG and TG are added to a protein essentially at the same time" means that the PG and TG are added during a sequence of steps for addition of raw materials. In commercial manufacturing steps, raw materials are usually added sequentially, that is, at first material A is added, then material B is added, then material C is added, and so on instead of mixing these materials in advance. In such a case, when PG and TG are added during the sequential steps for addition of raw materials, the method is categorized that "protein glutaminase and transglutaminase are added to a protein essentially at the same time" as the present invention. It is needless to say, of course, in a case where raw materials including PG and TG are mixed in advance and then added, the method is categorized as "added essentially at the same time".

Temperature needed for the reactions of both PG and TG generally ranges approximately from 3 to 60 degrees C and it will take about 1 minute to about 48 hours to progress the reactions. However, it may be better to spend about 5 minutes to about 24 hours at a temperature approximately, 5 to 50 degrees C. When adding PG and TG to a food, only PG and TG may be added to a material containing a protein as a substrate, or PG and TG may be added with other materials. Amount of PG and TG to be added can be varied according to a kind of protein to be modified, final product or an effect to be obtained. For example, a standard amount of TG to be added is 0.1 to 100 units per 1 gram-weight of protein in materials for food. On the other hand, a standard amount of PG to be added is 0.01 to 120 units per 1 gram-weight of protein in materials for food. These amounts to be added are mere estimations and not limited to the values as far as the effect of the present invention can be obtained.

According to the present invention, a ratio of PG to TG, both of which are to be acted on a protein, is important. In a case where an adding timing of PG to a protein is essentially the same as an adding timing of TG to the protein or before the TG acts on the protein, the protein can be modified when the ratio of protein glutaminase to transglutaminase both of which are added to the protein is PG:TG= 0.05 to 3:1 by activity and preferably PG:TG=0.05 to 2:1 because both of the PG and TG act on the protein in these range of the ratio. Or when the ratio of PG to TG is PG:TG= 0.01 to 0.7:1 by weight, preferably PG:TG= 0.01 to 0.4:1 and more preferably PG:TG= 0.01 to 0.3:1, the protein can be modified because both of the PG and TG act on the protein in these range of the ratio. When the ratio of PG to TG is greater than the ratio above indicated, an action of the PG becomes too large compared to that of TG and the effect of using them together cannot be obtained. On the other hand, when the ratio is smaller, an action of PG is too small to obtain the effect of using them together.

When the ratio of protein glutaminase to transglutaminase both of which are added to the protein is PG:TG= 0.2 to 3.0:1 by signal intensity of NMR and preferably PG:TG= 0.2 to 2.3:1, the protein can be modified because both of the PG and TG act on the protein regardless to the adding timings. When the ratio of PG to TG is greater than the ratio above, an action of the PG becomes too large compared to that of TG and the effect of using them together cannot be obtained. On the other hand, when the ratio is smaller, an action of PG is too small to obtain the effect of using them together.

The ratio of signal intensity of NMR of the present invention can be calculated by the following method. A substrate protein is treated by PG or TG in the presence of isotope labeled ($^{15}$N or $^{14}$N) ammonium salt to label a glutamine residue of the protein, and then an NMR signal of the labeled protein is measured and calculated. A kind of NMR measurement method is not limited and, for example, a correlation spectrum of $^1$H and $^{15}$N such as a HSQC spectrum may be measured for detecting a glutamine residue labeled with $^{15}$N. When two or more signals are obtained, the sum of signal intensity is calculated as the signal intensity. As a result, the signal intensity ratio is expressed as "the sum of signal intensity when the protein is acted by PG" / "the sum of signal intensity when the protein is acted by TG". The labeled compound may be an ammonium salt such as ammonium chloride and ammonium sulfate, and the like. When labeling the glutamine residue with $^{15}$N, an ammonium salt having $^{15}$N as ammonium nitrogen may be used and when labeling the glutamine residue with $^{14}$N, an ammonium salt having $^{14}$N as ammonium nitrogen may be used. A labeling method is such that a protein to be labeled and an ammonium salt are allowed to stand in an aqueous solvent at a pH ranging from about 3.0 to about 9.0 and preferably from about 4.0 to about 8.0 and at a temperature ranging from about 4 to about 65 degrees C and preferably from about 25 to about 60 degrees C. A reaction time is not particularly limited and may be about 30 seconds to 1 week and preferably about 1 minute to about 1 day. In this reaction, a concentration of the ammonium salt may be preferably more than about ten times to a concentration of a protein to be labeled and more preferably more than about 200 times. When a concentration of the protein to be labeled is from about 1 $\mu$M to about 40 mM, a concentration of the ammonium salt may be preferably from about 10 $\mu$M to about 10 M. The signal intensity ratio of the present invention can be measured for every kind of food containing protein because proteins contained in food such as $\alpha$-lactoglobulin, casein, soybean globulin, myosin, actomyosin, and the like can be labeled with isotope.

An example is explained that a protein is reacted with isotope-labeled ammonium chloride by treating with TG or PG. When $\alpha$-lactalbumin (referred to as "$\alpha$-La" hereinafter) is used as a substrate, a solution of the substrate (10 mg/ml of $\alpha$-La, 200 ml of $^{15}NH_4Cl$, 5% $D_2O$/20mM Tris-HCl (pH 7.0)) is prepared. Then TG or PG is added such that a ratio of substrate to enzyme becomes 1000:1 and incubated for 26.5 hours at 37 degrees C. The incubated solution is poured into an NMR sample tube and $^1H$-$^{15}N$ HSQC is measured using Avance 600 (Bruker Corporation), etc. Carboxyamide nitrogen is replaced with $^{15}N$ and two signals are observed as a pair per one chemical shift of $^{15}N$ because two atoms of $^1H$ are bonded with the $^{15}N$. The ratio of NMR signal intensity can be calculated by "the sum of signal intensity treated with PG" / "the sum of signal intensity treated with TG".

A protein that can be modified by the present invention is not particularly limited and, for example, milk protein, whey protein, soybean protein, wheat gluten, muscle protein, plasma, collagen, gelatin, and the like may be used. A mixture of two or more of these proteins may be also possible. A food according to the present invention is not limited by kinds of raw material or processed state as far as it contains the protein above mentioned modified by an use of PG and TG together. A food of processed state such as; for example, sterilized state, defatted state, diluted state, condensed state, dried state, and the like is included within the scope of the present invention.

Next, an enzyme preparation of the present invention will be explained. An enzyme preparation of the present invention for modifying a protein has a condition that a mixing ratio of transglutaminase to protein glutaminase as essential components is in the range from PG:TG=0.05 to 3:1 by activity and preferably from PG:TG=0.05 to 2:1; in the range from PG:TG=0.01 to 0.7:1 by weight, preferably from PG:TG=0.01 to 0.4:1 and more preferably from PG:TG=0.01 to 0.3:1; or in the range of PG:TG=0.2 to 3.0:1 by NMR signal intensity and preferably from PG:TG=0.2 to 2.3:1. Any component that is generally used in the present field other than PG and TG such as lactose, sucrose, maltitol, sorbitol, dextrin, branched dextrin, cyclodextrin, starch, polysaccharides, gum, pectin, and the like may be formulated. An animal protein or a vegetable protein such as a soybean protein, wheat protein, etc. can be also formulated. Moreover, an inorganic salt physiologically acceptable such as a sodium bicarbonate, sodium citrate, sodium phosphate, sodium chloride, potassium chloride, etc. can be formulated when necessary in the enzyme preparation of the present invention. Moreover, a seasoning, sugar, spice, coloring agent, color coupler, organic salt such as an ascorbic acid and its salt, or an emulsifier, oil and fat can be formulated as necessary.

The present invention includes a simple method to find an optimum quantitative adding ratio of protein glutaminase to transglutaminase. The method is that, as already described, a protein is treated with PG and TG separately in the presence of an isotope labeled ammonium salt, to label a functional group of a glutamine residue of the protein with the isotope, and the NMR signal intensity is measured. The optimum quantitative adding ratio of PG to TG can be established by finding a condition that the NMR signal intensity ratio of protein glutaminase to transglutaminase becomes from protein glutaminase:transglutaminase= 0.2 to 3.0:1 and preferably from 0.2 to 2.3:1. The optimum range of the NMR signal intensity ratio of PG to TG ranges from protein glutaminase:transglutaminase=0.2 to 3.0:1 and preferably ranges from 0.2 to 2.3:1 without depending on kinds of substrate proteins. Because the NMR signal intensity ratio correlates to the optimum adding ratio (activity ratio or weight ratio) of PG to TG for each of the substrate protein, the optimum adding ratio (activity ratio or weight ratio) of PG to TG can be easily found without repeating trial and error of many experiments.

The present invention will be explained in detail with following experimental examples and examples; however, the scope of the present invention is not limited to these examples.

EXPERIMENTAL EXAMPLE 1

An $\alpha$-lactalbumin (referred to as "$\alpha$-La" hereinafter) (Sigma) was used as a substrate. A solution of the substrate (10 mg/ml of $\alpha$-La, 200 ml of $^{15}NH_4Cl$, 5% $D_2O$/20mM Tris-HCl (pH 7.0)) was prepared and then TG (purified enzyme from "Activa" TG, Ajinomoto Co., Inc.) or PG (prepared from Chryseobacterium described in Patent Document 1) was added such that a ratio of substrate to enzyme became 1000:1 and incubated for 26.5 hours at 37 degrees C. The incubated solution was poured into an NMR sample tube and $^1H$-$^{15}N$ HSQC was measured using NMR (Avance 600, Bruker Corporation). The result of the $^1H$-$^{15}N$ HSQC measurement after 26.5 hours is shown in Fig. 1. When carboxyamide nitrogen is replaced with $^{15}$N, two signals are observed as a pair per one chemical shift of $^{15}$N because two atoms of $^1$H are bonded with the [15]N. It can be seen that a part of nitrogen atoms of carboxyamides of glutamine residues in α-La is labeled with $^{15}$N and succeeded in exhibiting $^{15}$N labeling by PG as shown in Fig. 1. Therefore, it was proved that PG can also cause reaction between ammonium chloride and glutamine residue in addition to deamidation reaction likewise as TG. NMR signal intensity is indicated as the sum of areas of oval spots in Fig. 1 and the larger the area is, the larger the amount of the reaction is.

EXAMPLE 1

TG or PG as explained in Experimental Example 1 was added independently to a milk on the market, 0.2 M [15]NH$_4$Cl and 5% D$_2$O, and signal intensity at each added concentration was measured. TG was added such that a ratio of substrate to enzyme (S/E ratio) was 7400/1 by weight. On the other hand, PG was added by 0.002 to 5 times of TG by weight. A relative activity of the TG was 26 units per 1 mg of enzyme protein and a relative activity of the PG was 120 units per 1 mg of enzyme protein. The solution containing the enzyme was then incubated for 3 hours at 37 degrees C, and then poured into an NMR sample tube and measured by $^1$H-$^{15}$N HSQC using NMR described in Experimental Example 1. The amount of added PG was indicated by weight ratio (PG/TG) of enzymes and activity ratio (PG/TG) against the amount of TG added. Signal intensity ratio (PG/TG) corresponding to each ratio is shown in Table 1.
[Table 1]

| TG | PG | Weight ratio of ratio of enzymes (PG/TG) | Activity ratio (PG/TG) | Signal intensity | Signal intensity ratio (PG/TG) |
|---|---|---|---|---|---|
| 2.5 μg | - | - | - | 92.7 | - |
| - | 12.5 μg | 5 | 23 | 474 | 5.11 |
| - | 2.5 μg | 1 | 4.6 | 308.1 | 3.32 |
| - | 0.5 μg | 0.2 | 0.92 | 147 | 1.58 |
| - | 0.25 μg | 0.1 | 0.46 | 93.5 | 1 |
| - | 0.025 μg | 0.01 | 0.046 | 20.1 | 0.216 |

Yoghurt was made from milk on the market. 400 g of milk on the market was heated to 50 degrees C and added with TG and PG described in Experimental Example 1 by the amounts shown in Table 2. A relative activity of the TG was 26 units per 1 mg of enzyme protein and a relative activity of the PG was 120 units per 1 mg of enzyme protein. After enzyme reaction of 90 minutes at 50 degrees C, it was heated in a boiling bath with stirring. As soon as the temperature reached 90 degrees C, it was soaked in water with ice to cool to 45 degrees C. 20 g (5% weight of original material) of starter (Bulgaria yoghurt, Meiji Dairies Corporation) was added, stirred completely and divided to each sample cup by 40 g. The cups were covered with aluminum foil and fermented approximately 3 to 4 hours at 38 degrees C until pH dropped to 4.5. They were reserved in low temperature (4 degrees C) and evaluated next day by a physical property test and a sensory test.
A breaking stress as a physical property was measured using a texture analyzer (Eko Instruments). A cylindrical plunger of 10 mm in diameter was used and a breaking speed was set at 6 cm/min (1 mm/sec). The result is shown in Table 3. A breaking stress of a comparative product T, in which only TG was added, increased remarkably compared with a control sample. The result is attributed to an effect of linkage of milk protein by the TG. Products 1 to 4 of the present invention also showed relatively high breaking stresses compared with the control sample, which means that an effect to increase breaking stress by TG was maintained. However, because a breaking stress of a comparative product T-P does not differ from that of comparative product P, it was confirmed that the TG reaction was almost ceased by PG and therefore the effect by TG was not observed.
[Table 2]

| | enzyme | TG (mg) | PG (mg) | Enzyme weight ratio (PG/TG) | Enzyme activity ratio (PG/TG) | Signal intensity ratio (PG/TG) |
|---|---|---|---|---|---|---|
| Control sample | - | - | - | - | - | - |

(continued)

| | enzyme | TG (mg) | PG (mg) | Enzyme weight ratio (PG/TG) | Enzyme activity ratio (PG/TG) | Signal intensity ratio (PG/TG) |
|---|---|---|---|---|---|---|
| Product 1 of the present invention | TG, PG | 1.15 | 0.0115 | 0.01 | 0.046 | 0.216 |
| Product 2 of the present invention | TG, PG | 1.15 | 0.023 | 0.02 | 0.092 | Not measured |
| Product 3 of the present invention | TG, PG | 1.15 | 0.115 | 0.1 | 0.46 | 1 |
| Product 4 of the present invention | TG, PG | 1.15 | 0.23 | 0.2 | 0.92 | 1.58 |
| Comparative product T | TG | 1.15 | - | - | - | - |
| Comparative product P | PG | - | 1.15 | - | - | - |
| Comparative product T-P | TG, PG | 1.15 | 1.15 | 1 | 4.6 | 3.32 |

[Table 3]

| | Breaking stress (g) |
|---|---|
| Control sample | 18.63 |
| Product 1 of the present invention | 33.88 |
| Product 2 of the present invention | 31.74 |
| Product 3 of the present invention | 24.68 |
| Product 4 of the present invention | 20.51 |
| Comparative product T | 32.17 |
| Comparative product P | 12.91 |
| Comparative product T-P | 12.44 |

Hardness was evaluated as a sensory test by 6 panels. The hardness of the control sample is set as 0 point, and each product was scored by +5 points as hardest and -5 points as softest and an average point for each product was calculated. The result is shown in Table 4. The comparative product T, in which only TG was added, increased its hardness remarkably compared with the control sample. Products 1 to 4 of the present invention also kept increased hardness obtained by TG. However, the hardness of yoghurt of comparative product T-P was softened as the comparative product P, which means that the TG reaction was almost ceased by PG and therefore the effect by TG was not observed. The result roughly corresponds to the result of the physical property test explained above. The overall evaluation of oral sensation preference indicated that the products 1 to 4 of the present invention obtained favorable result, that is, the products kept an effect of increased hardness of yoghurt by TG and, on the other hand, smoothness was improved than the case when TG only was added. This means that an effect of using PG and TG together was confirmed.

[Table 4]

|  | Average hardness (n=6) | Comment | Overall evaluation (point) |
|---|---|---|---|
| Control sample | 0 | - | 2.5 |
| Product 1 of the present invention | 3.7 | hard, smooth texture | 3.1 |
| Product 2 of the present invention | 3.8 | hard, smooth texture | 3.2 |
| Product 3 of the present invention | 3.2 | very thick oral very thick oral sensation, creamy | 4.8 |
| Product 4 of the present invention | 2.6 | thick oral sensation creamy | 4.2 |
| Comparative product T | 3.2 | hard and rough | 1.9 |
| Comparative product P | -2 | too soft | 2 |
| Comparative product T-P | -1.2 | too soft | 2.4 |

1 point: not favorable
2 points: not so favorable
3 points: fair
4 points: favorable
5 points: very favorable

EXAMPLE 2

Skim milk powder (low heat-type, milk protein 35%, Yotsuba Co., Ltd.) was added by 0.85% to Takanashi Milk Products Co. Ltd.'s low fat milk (milk protein 3.3%, milk fat 1.0%) to adjust the milk protein concentration to 3.6% and dissolved at 55 degrees C by heating to prepare raw material milk for yoghurt. The raw material milk was heated in a boiling bath, kept in two minutes after reached 95 degrees C and then cooled in an ice bath immediately. When the raw material milk reached 47 degrees C, a lactic acid bacteria starter (Yo-Flex, DVS YC-370, Christian Hansen) was added by 0.006%, and TG and PG were added according to Table 5 and stirred well. Then it was divided into plastic cups by specified amount and fermented in an incubator at 44 degrees C until the pH reached 4.5 to 4.6. It took approximately 4 to 5 hours from the beginning to the end of fermentation. After fermentation, the products were preserved in a refrigerator at 5 degrees C. Next day, an amount of syneresis on the surface of the obtained set-type yoghurt was observed. Physical properties of the yoghurt were evaluated using a texture analyzer (Stable Macro System, Ltd.). A sensory test was performed by 5 trained panels. A breaking stress and an adhesion area of the yoghurt were measured at a condition for the physical property measurement of 1 mm/sec velocity, plate plunger of 10 mm diameter and 10% of compression. The inventors have already confirmed that the breaking stress highly correlates to hardness of yoghurt and the adhesion area highly correlates to texture of creaminess of yoghurt. The result is shown in Table 6.
[Table 5]

|  | TG(u/gp) | PG(u/gp) | Activity ratio (PG/TG) | Weight ratio (PG/TG) |
|---|---|---|---|---|
| Control sample | 0 | 0 | - | - |
| Comparative product 1 | 0.5 | 0 | - | - |
| Comparative product 2 | 0 | 0.5 | - | - |
| Comparative product 3 | 0 | 1 | - | - |
| Product 1 of the present invention | 0.5 | 0.5 | 1 | 0.22 |
| Product 2 of the present invention | 0.5 | 1 | 2 | 0.44 |

[Table 6]

|  | syneresis on surface | breaking stress (g) | adhesion area (g*ε) | sensory test (n=5) | overall evaluation |
|---|---|---|---|---|---|
| Control ample | large | 19.1 | 3.26 | rough surface, sloppy and soft texture | × |
| Comparative product 1 | none | 24.3 | 3.93 | glossy surface, hard and crumbly (like kanten), but sloppy | × |
| Comparative product 2 | large | 15.8 | 4.31 | rough surface, soft and smooth | △ |
| Comparative product 3 | fairly large | 14.3 | 4.52 | fairly rough surface, very soft and smooth, of less body | △ |
| Product 1 of the present invention | none | 28.6 | 4.41 | glossy surface, feel body and smooth | ○ |
| Product 2 of the present invention | none | 21.1 | 5.2 | glossy surface, feel body, very smooth and creamy | ○ |

A large amount of syneresis was observed on the surfaces of the control sample and comparative product 2 and a fairly large amount of syneresis was observed on the comparative product 3. No syneresis was observed on the surfaces of the comparative product 1 and products 1 and 2 of the present invention and the surfaces of these products were glossy. The physical property measurements and the sensory evaluation showed that the comparative product 1 had a hard and crumbly texture like an agar gel and was sloppy in a mouth. The comparative products 2 and 3 decreased their hardness than the control sample and increased their adhesiveness. The sensory test also caused a comment that they were smooth but soft and of less body. On the other hand, the products 1 and 2 of the present invention increased both of hardness and adhesiveness and they were of body, smooth and creamy yoghurt. As an overall evaluation including appearance, physical property and oral sensation, the products 1 and 2 of the present invention are the most favorable yoghurts, indicating that an improvement of oral sensation of set-type low fat yoghurt is possible by adding TG and PG in an appropriate balanced ratio of blending.

EXAMPLE 3

Skim milk powder (low heat-type, milk protein 35%, Yotsuba Co., Ltd.) was added to Takanashi Milk Products Co. Ltd.'s low fat milk (milk protein 3.3%, milk fat 1.0%,) to adjust the milk protein concentration to 3.94% and dissolved at 55 degrees C by heating to prepare raw material milk for yoghurt. The raw material milk was heated in a boiling bath, kept in two minutes after reached 95 degrees C and then cooled in an ice bath immediately. When the raw material milk reached 47 degrees C, a lactic acid bacterium starter (Yo-Flex, DVS YC-370, Christian Hansen) was added by 0.006% and TG and PG were added according to Table 7 and stirred well. Then it was divided into stainless cups by specified amount and fermented in an incubator at 44 degrees C until the pH reached 4.5 to 4.6. After fermentation, the products were cooled in a refrigerator at 5 degrees C and filtered through a filter of 216 μm meshes to make stirred yoghurt. The resultant stirred yoghurt was preserved at 5 degrees C and a change of appearance and oral sensation during preservation were evaluated. A physical property of the yoghurt was measured by a dynamic viscoelasticity measuring device (Rheostress RS1, HAAKE). A sensory test was performed by 3 trained panels. A measurement condition for physical property of the yoghurt was programmed, in which a shearing speed of a corn plate of 6 cm diameter was increased from zero to 100 (1/s) during 300 seconds and then the shearing speed was decreased from 100 to zero (1/s) during the same period of time. The measurement temperature was 10 degrees C and a viscosity at the shearing velocity 100 (1/s) was recorded. The result is shown in Table 8.

[Table 7]

|  | TG(u/gp) | PG(u/gp) | Activity ratio (PG/TG) | Weight ratio (PG/TG) |
|---|---|---|---|---|
| Control sample | 0 | 0 | - | - |
| Comparative product 1 | 0.6 | 0 | - | - |
| Comparative product 2 | 0 | 0.6 | - | - |
| Product 1 of the present invention | 0.6 | 0.1 | 0.17 | 0.037 |
| Product 2 of the present invention | 0.6 | 0.3 | 0.5 | 0.11 |
| Product 3 of the present invention | 0.6 | 0.6 | 1 | 0.22 |
| Product 4 of the present invention | 0.6 | 1.0 | 1.7 | 0.37 |

[Table 8]

|  | syneresis on surface | sensory test immediately after trial preparation (n=5) | Viscosity (mPεs) | lump after long preservation | oral sensation after long preservation | overall evaluation |
|---|---|---|---|---|---|---|
| Control sample | Large | rough, sloppy and soft texture | 98.7 | None | not changed | × |
| Comparative product 1 | None | rough and of body | 160.9 | Large | texture like sand | × |
| Comparative product 2 | Small | glossy surface, smooth but sloppy and soft | 99.3 | None | Not changed | △ |
| Product 1 present invention | None | glossy surface, of body, fairly smooth | 161.5 | Fairly small | decreased texture like sand | ○ |
| Product 2 of the present invention | None | glossy surface, of body, very smooth | 157.8 | Small | no texture like sand | ◎ |
| Product 3 of the present invention | None | glossy surface, fairly of body, very smooth | 129.0 | None | no texture like sand | ○ |
| Product 4 of the present invention | None | glossy surface, of body than reference, very smooth | 116.5 | none | no texture like sand | ○ |

Before preparation of the stirred yoghurts, a large amount of syneresis and small amount of syneresis was observed on the control sample and the comparative product 2, respectively, and almost no syneresis was observed on the comparative product 1 and products 1 to 4 of the present invention. According to the sensory test immediately after preparation of the stirred yoghurt, the control sample had rough, sloppy and soft texture. The comparative product 1 had body but felt rough, and the comparative product 2 had a glossy surface and felt smooth but sloppy and soft. The products 1 to 4 of the present invention had glossy surfaces and had smoothness as well as body. The results of the viscosity measurement well correspond to the results of the sensory tests. That is, the viscosities of the products 1 to 4 of the present invention were apparently high compared with that of the control sample or the comparative product 2, which proved that the products 1 to 4 of the present invention have obtained body. The change of appearances and oral sensation after three weeks' preservation in a refrigerator was that lumps were produced in the comparative product 1 and rough texture like sand was strongly sensed. However, the texture was improved in the product 1 of the present invention and almost eliminated in the products 2 to 4 of the present invention. According to the results explained above in all, the products 1 to 4 of the present invention were evaluated to be apparently superior to the control sample and comparative products 1 and 2 by the points of the physical property and oral sensation (after preservation).

As explained above, the yoghurt added only by TG (comparative product 1) produces body compared with yoghurt added no TG but the quality degradation during preservation may be a problem. On the other hand, the yoghurt added only by PG (comparative product 2) feels smooth but the sloppy texture without body may be a problem. However, it was proved that the yoghurt added by TG and PG at a specified ratio (products 1 to 4 of the present invention) could improve quality of the yoghurt by rendering glossy surface and smoothness as well as eliminating above problems.

EXAMPLE 4

TG or PG described in EXPERIMENTAL EXAMPLE 1 were solely added to soya milk on the market (not modified, final protein content 4.8%, Taishi-Food Inc.), 0.2M $^{15}NH_4Cl$ and 5% $D_2O$ and mixed well. The TG was added such that a ratio of substrate/enzyme (S/E ratio) was 6000/1 by weight. The PG was added by 0.01 to 1 time of the amount of the TG. The solution was incubated for 1 hour and 15 minutes at 37 degrees C and then the solution was poured into an NMR sample tube and $^1H$-$^{15}N$ HSQC was measured by the NMR described in EXPERIMENTAL EXAMPLE 1. Calculated results of signal intensity ratio of PG/TG at each enzyme weight ratio (PG/TG) and activity ratio (PG/TG) as is explained in EXPERIMENTAL EXAMPLE 1 are shown in Table 9.

[Table 9]

| TG | PG | Enzyme weight ratio (PG/TG) | Activity ratio (PG/TG) | Signal intensity | Signal intensity ratio (PG/TG) |
|---|---|---|---|---|---|
| 4 μ g | - | - | - | 60.96 | |
| - | 4 μ g | 1 | 4.6 | 373.48 | 2.52 |
| - | 1 μ g | 0.25 | 1.15 | 364.31 | 2.26 |
| - | 0.4 μ g | 0.1 | 0.46 | 208.87 | 1.29 |
| - | 0.2 μ g | 0.05 | 0.23 | 113.22 | 0.703 |
| - | 0.04 μ g | 0.01 | 0.04 | 23.421 | 0.145 |

Bean curds added by TG and PG by the ratio described in Table 10 were prepared. A solidification container containing 10 g of 30% bittern solution (containing 3g of bittern, Ako Kasei Co., Ltd.) and a large stainless cup containing 1 kg of soya milk on the market above were heated to 55 degrees C in a hot water bath. An enzyme solution was added into the soya milk and stirred. The soya milk was poured immediately into the solidification container containing the bittern vigorously and a stirring plate was moved up and down by four times. The container was covered with a cap and then transferred into an incubator and allowed to stand for 50 minutes at 55 degrees C. After that the bean curd was transferred into water in a vat and kept 30 minutes to 1 hour. The bean curd was divided and put into curd containers and weighed the content. Water was poured into the containers up to a level flush with the bean curd and heat-sealed with films. They were put into a thermostat water bath at 85 degrees C and kept for 45 minutes (secondary heating) and then cooled at first in a flowing water and then in an iced bath, and reserved in cold storage. Next day water was drained and the content in the container was weighed and physical property test and sensory test were performed.

[Table 10]

|  | Enzyme | TG (mg) | PG (mg) | Enzyme weight ratio (PG/TG) | Enzyme activity ratio (PG/TG) | Signal intensity ratio (PG/TG) |
|---|---|---|---|---|---|---|
| Control sample | - | - | - | - | - | - |
| Comparative product (T1) | TG | 0.15 | - | - | - | - |
| Comparative product (T2) | TG | 0.75 | - | - | - | - |
| Comparative product (P1) | PG | - | 0.036 | - | - | - |
| Comparative product (P2) | PG | - | 0.18 | - | - | - |
| Comparative product (T-P) | TG, PG | 0.15 | 0.18 | 1.2 | 5.5 | Not measured |
| Product 1 of the present invention | TG, PG | 0.15 | 0.038 | 0.25 | 1.15 | 2.26 |
| Product 2 of the present invention | TG, PG | 0.75 | 0.038 | 0.05 | 0.23 | 0.703 |
| Product 3 of the present invention | TG, PG | 0.75 | 0.19 | 0.25 | 1.15 | 2.26 |

A breaking test was performed as a physical property test using a rheometer (Fudou Kougyou Inc.). The plunger was disc-shaped whose size was 5 mm in diameter and a breaking speed was 6 cm/min (1 mm/sec). A sensory test was performed by 5 panels and smoothness and hardness were evaluated such that a product without enzyme scored zero point, and each product was evaluated by 7 grades between ±3 points and an average was calculated. The result is shown in Table 11. The comparative products T1 and T2 in which TG was added increased their breaking stresses compared with the control sample. On the other hand the comparative products P1 and P2 in which PG was added decreased their breaking stresses compared with the control sample. A breaking stress of the product 1 of the present invention was almost equivalent degree to that of the comparative product T1 in which the same amount of TG was added and the texture of the product 1 of the present invention changed smoother. A breaking stress of the product 2 of the present invention slightly decreased compared with the comparative product T2 in which the same amount of TG was added; however, the breaking stress was still large compared with the control sample and smoothness was also given. A breaking stress of the product 3 of the present invention decreased compared with the comparative product T2 in which the same amount of TG was added; however, the breaking stress was larger than that of the comparative product P2 in which the same amount of PG was added and therefore, an effect of combined use of TG was confirmed. The comparative product T-P was as smooth as that of comparative product P1 or P2 and a hardening effect of the TG reaction was not observed. The result means that the TG reaction was almost ceased by PG. The sensory test showed that the hardening effect by TG was observed in the products 1 to 3 of the present invention and their textures were improved compared with the case only TG was added. As an overall result, a preferable texture was obtained for the products of the present invention, which means that the effect of using PG and TG together was confirmed.

[Table 11]

|  | Breaking stress (g) | Comments | Overall evaluation (point) |
|---|---|---|---|
| Control sample | 33.9 | - | 2.5 |
| Comparative product (T1) | 38.4 | hard | 3.2 |
| Comparative product (T2) | 54.1 | very hard | 3.5 |

(continued)

|  | Breaking stress (g) | Comments | Overall evaluation (point) |
|---|---|---|---|
| Comparative product (P1) | 28.2 | soft | 2.4 |
| Comparative product | 17.9 | very soft, fragile (crumbly) | 2 |
| Comparative product (T-P) | 21.5 | too soft | 2.7 |
| Product 1 of the present invention | 36.6 | very smooth, creamy | 4.1 |
| Product 2 of the present invention | 49.2 | hard and smooth texture | 4.8 |
| Product 3 of the present invention | 34.1 | very smooth, creamy | 4.2 |

1 point: not favorable
2 points: not so favorable
3 points: fair
4 points: favorable
5 points: very favorable

EXAMPLE 5

Japanese udon noodles and Chinese noodles were prepared according to the formulation and trial procedures shown in Table 12. Transglutaminase and protein glutaminase were dissolved in water when added. The amounts of enzymes added in the udon noodles and Chinese noodles are shown in Table 13. The udon noodles were boiled for 20 minutes in hot water 15 times the amount of 100 g of the noodle and the oral sensation was evaluated by trained panels (n=5). The Chinese noodles were evaluated as the udon noodles except for the boiling time of 5 minutes. Hardness, stickiness and smoothness were evaluated by the standard of a control sample (apparently decreased: $\times\times$, slightly decreased: $\times$, equal: $\triangle$, slightly increased: $\bigcirc$, apparently increased: $\circledcirc$). Because the results of the evaluation for udon noodles and Chinese noodles had almost the same tendency, they are shown together in Table 14. When the ratio of PG/TG by weight was 0.01, 0.05 or 0.2 (corresponding to product 1, 2 or 3 of the present invention, respectively), the oral sensation was well balanced with hardness, stickiness and smoothness for both of the noodles. It was proved that these ratios were also appropriate ratios for noodles to obtain favorable effects of both transglutaminase and protein glutaminase by using them together.
[Table 12]

| Udon noodles | formulation | Wheat 100 portions, salt 3 portions, water 40 portions |
|---|---|---|
|  | procedure | First rolling, scale 2.5 → combined rolling, scale 2.5 → rolling, scale 2.3 → rolling, scale 2.1 → rolling, scale 1.7 → rolling, scale 1.5 → ferment, 1h → cutting (angular blade #12) |
| Chinese noodles | formulation | Wheat 100 portions, salt 1 portion, lye water A 1 portion, food coloring 0.1 portion, water 36 portions |
|  | procedure | First rolling, scale 1.5 → combined rolling, scale 1.5 → rolling, scale 1.3 → rolling, scale 1.1 → rolling, scale 0.7 → rolling, scale 0.5 → ferment, 1h → cutting (angular blade #22) |

[Table 13]

| Category | Enzyme | TG (mg) | PG (mg) | Enzyme weight ratio (PG/TG) | Enzyme activity ratio (PG/TG) |
|---|---|---|---|---|---|
| Control sample | - | 0 | 0 | - | - |
| Comparative product 1 | PG | 0 | 0.1 | - | - |
| Comparative product 2 | PG | 0 | 0.5 | - | - |

(continued)

| Category | Enzyme | TG (mg) | PG (mg) | Enzyme weight ratio (PG/TG) | Enzyme activity ratio (PG/TG) |
|---|---|---|---|---|---|
| Comparative product 3 | PG | 0 | 2 | - | - |
| Comparative product 4 | TG | 10 | 0 | - | - |
| Product 1 of the present invention1 | TG, PG | 10 | 0.1 | 0.01 | 0.046 |
| Product 2 of the present invention1 | TG, PG | 10 | 0.5 | 0.05 | 0.23 |
| Product 3 of the present invention1 | TG, PG | 10 | 2 | 0.2 | 0.92 |

[Table 14]

| Evaluation category | Hardness | Stickiness | Smoothness | Overall evaluation |
|---|---|---|---|---|
| Control sample | standard | standard | standard | standard |
| Comparative product 1 | × | ○ | ○ | △ |
| Comparative product 2 | × | ◎ | ◎ | △ |
| Comparative product 3 | × × | ◎ | ◎ | △ |
| Comparative product 4 | ◎ | △ | △ | △ |
| Product 1 of the present invention 1 | ◎ | ○ | ○ | ○ |
| Product 2 of the present invention | ○ | ◎ | ◎ | ◎ |
| Product 3 of the present invention 1 | ○ | ◎ | ◎ | ◎ |
| × ×: apparently decreased, ×: slightly decreased, △: equal, ○: slightly increased, ◎: apparently increased | | | | |

INDUSTRIAL APPLICABILITY

According to the present invention, different protein- modifying effects from that of the case where TG or PG alone is used can be obtained and a food containing protein that has new characteristics can be produced. In addition, an optimum condition to treat substrate by TG and PG can be easily established. Therefore, the present invention is greatly useful for the food manufacturing field.
The disclosures of Patent Documents and References are incorporated herein in their entirety by reference thereto. The modes or examples of the invention can be modified or adjusted within the ambit of the entire disclosures (including the claims) of the present invention, and based on the technical scope of the invention. Moreover, various combinations or selections of the various disclosed elements are possible within the scope of the claims of the present invention.

**Claims**

1. A method of modifying a protein by treating the protein with both of protein glutaminase and transglutaminase, wherein a timing of adding protein glutaminase to a protein is essentially the same as a timing of adding transglutaminase to the protein or before the transglutaminse acts on the protein.

2. The method of claim 1, wherein a ratio by activity of the protein glutaminase to the transglutaminase, both of which are to be added to the protein, is the protein glutaminase: the transglutaminase =0.05 to 3:1.

3. The method of claim 1 or 2, wherein a ratio by weight of the protein glutaminase to the transglutaminase, both of which are to be added to the protein, is the protein glutaminase: the transglutaminase =0.01 to 0.7:1.

4. The method of one of claims 1 to 3, wherein a ratio by NMR signal intensity of the protein glutaminase to the transglutaminase, both of which are to be added to the protein, is the protein glutaminase: the transglutaminase = 0.2 to 3.0:1.

5. The method of one of claims 1 to 4, wherein the protein is one or more selected from a group consisting of a milk protein, whey protein, soybean protein, wheat gluten, plasma, muscle protein, collagen and gelatin.

6. A food comprising a protein modified by the method of one of claims 1 to 5.

7. An enzyme preparation for modifying a protein comprising protein glutaminase and transglutaminase, wherein a ratio by activity of the protein glutaminase to the transglutaminase in the enzyme preparation is the protein glutaminase: the transglutaminase = 0.05 to 3:1.

8. An enzyme preparation for modifying a protein comprising protein glutaminase and transglutaminase, wherein a ratio by weight of the protein glutaminase to the transglutaminase in the enzyme preparation is the protein glutaminase: the transglutaminase =0.01 to 0.7:1.

9. A enzyme preparation for modifying a protein comprising protein glutaminase and transglutaminase, wherein a ratio by NMR signal intensity of the protein glutaminase to the transglutaminase in the enzyme preparation is the protein glutaminase: the transglutaminase =0.2 to 3.0:1.

10. A method of establishing an optimum ratio of protein glutaminase to transglutaminase, both of which are to be added to a protein, comprising:

treating a protein with transglutaminase and protein glutaminase separately under presence of an isotope-labeled ammonium salt,
labeling a functional group of glutamine residue of the protein with the isotope, and
measuring an NMR signal intensity of the protein.

FIG. 1

**INTERNATIONAL SEARCH REPORT**

| International application No. |
| --- |
| PCT/JP2009/054792 |

A. CLASSIFICATION OF SUBJECT MATTER
*C12P21/02*(2006.01)i, *C12N9/10*(2006.01)i, *C12Q1/48*(2006.01)i, *C12Q1/52* (2006.01)i

According to International Patent Classification (IPC) or to both national classification and IPC

B. FIELDS SEARCHED

Minimum documentation searched (classification system followed by classification symbols)
C12P21/02, C12N9/10, C12Q1/48, C12Q1/52

Documentation searched other than minimum documentation to the extent that such documents are included in the fields searched

Electronic data base consulted during the international search (name of data base and, where practicable, search terms used)
BIOSIS/MEDLINE/WPIDS(STN), JSTPlus(JDreamII), JST7580(JDreamII)

C. DOCUMENTS CONSIDERED TO BE RELEVANT

| Category* | Citation of document, with indication, where appropriate, of the relevant passages | Relevant to claim No. |
| --- | --- | --- |
| Y/A | JP 2000-050887 A (Amano Enzyme Inc.), 22 February, 2000 (22.02.00), & EP 0986829 A1 & US 6251652 A1 | 1-9/10 |
| Y/A | WO 2006/075772 A1 (AJINOMOTO CO., INC.), 20 July, 2006 (20.07.06), & EP 1839491 A1 & US 2007/0254065 A1 | 1-9/10 |
| Y/A | WO 2006/075771 A1 (AJINOMOTO CO., INC.), 20 July, 2006 (20.07.06), & EP 1836907 A1 & US 2007/0254066 A1 | 1-9/10 |
| A | JP 2002-332295 A (Ajinomoto Co., Inc.), 22 November, 2002 (22.11.02), & EP 1156330 A1 & US 2001/0044127 A1 | 1-10 |

| ☒ | Further documents are listed in the continuation of Box C. | ☐ | See patent family annex. |

| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search 08 May, 2009 (08.05.09) | Date of mailing of the international search report 19 May, 2009 (19.05.09) |
| --- | --- |
| Name and mailing address of the ISA/ Japanese Patent Office | Authorized officer |
| Facsimile No. | Telephone No. |

Form PCT/ISA/210 (second sheet) (April 2007)

| **INTERNATIONAL SEARCH REPORT** | International application No. |
|---|---|
| | PCT/JP2009/054792 |

C (Continuation).    DOCUMENTS CONSIDERED TO BE RELEVANT

| Category* | Citation of document, with indication, where appropriate, of the relevant passages | Relevant to claim No. |
|---|---|---|
| A | Yasuki MATSUMURA et al., "Shinki na Tanpakushitsu Datsu Amido Koso · Protein Glutaminase no Shokuhin Tanpakushitsu ni Taisuru Sayo", Shokuhin Kako Gijutsu, 2002, Vol.22, No.2, pages 11 to 18 | 1-10 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008066765 A
- JP 2008294890 A
- JP P200050887 A
- JP P2001218590 A
- JP P2002332295 A

- JP S6427471 A
- JP H0150382 B
- JP H01300889 A
- JP H06225775 A
- JP H0723737 A

**Non-patent literature cited in the description**

- **Yamaguchi et al.** *Eur.J.Biochem.,* 2001, vol. 268, 1410-1412
- Newest Technology and Application of Food Enzyme Chemistry-Prospect of Proteomics. CMC Publishing Co, 146-153
- **Y.S.Gu et al.** *J. Agric. Food Chem.,* 2001, vol. 49, 5999-6005

- **Phillips, M. A. et al.** *Proc. Natl. Acad. Sci. U.S.A.,* 1990, vol. 87, 9333
- **Ichinose, A. et al.** *Biochemistry,* 1990, vol. 25, 6900
- *Bulletin of Japanese Society of Fisheries Science,* 1990, vol. 56, 125-132